# EUROPEAN PATENT APPLICATION

(11) **EP 1 787 604 A1**
(43) Date of publication of application: **23.05.2007**
(21) Application number: 05405640.3
(22) Date of filing: 16.11.2005
(51) Int. Cl.: A61F 2/44

(54) **Implant for sealing and/or healing a defect in an annulus of an intervertebral disc**

(71) Applicant: Tissupor AG, 9001 Gallen (CH); Universität Bern, 3012 Bern (CH)
(72) Inventor: Ferguson, Stephen, 3251 Ruppoldsried (CH); Aebi, Max, 2503 Biel (CH); Bischoff, Bernhard, 9000 St. Gallen (CH); Steffen, Thomas, 3014 Bern (CH); Stoyanov, Jivko, 3004 Bern (CH); Billia, Mario F., 9542 Münchwilen (CH)
(74) Representative: Liebetanz, Michael

(57) **Abstract**

An implant for sealing and/or healing a defect in an annulus, comprising an area intended to encompass the defect. The implant is textile strip (6) that comprises a middle area (15) which is intended to encompass the defect and which is an oriented low-density embroidered structure for guided tissue ingrowth and reinforcement and end areas (14) adjacent to the middle area which are a high-density embroidered structure.

## Description

### Field of the invention

The invention relates to an implant for sealing and/or healing a defect in an intervertebral disc and in particular to a textile strip for sealing and/or healing a defect in the annulus fibrosus.

### Prior Art

Intervertebral discs lie between adjacent vertebral bodies in a spinal column. The intervertebral disc consists of a peripheral annulus fibrosus that surrounds the central nucleus pulposus. If someone suffers from a herniated intervertebral disc, the surrounding annulus fibrosus shows radial or circumferential fissures, tears or ruptures.

Many methods and devices to repair such a defect or to replace the whole intervertebral disc with a prothesis are well known by prior art. EP 991 379 shows in its Fig. 6 the use of a fabric to be attached to the artificial disc and the adjacent vertebrae.

WO 03/051239 discloses a method and an apparatus for sealing such a defect using a plug. A drawback of this invention is that the method is very complicated, since the insertion of such a plug mandates an extensive surgery.

US 2004/1,111,136 discloses a further device for diagnosis or treatment of a fissure in the intervertebral disc. A sealant can be injected via a catheter, thereby sealing the fissure from the inside. This method is rather complicated, as the annulus fibrosus is sealed from the inside.

Another method is the replacement of the whole intervertebral disc with a surgical implant, as disclosed for example by EP 0 991 379. Replacements are well known by prior art, but the surgeries are destructive to surrounding tissues, the recovery for the patient takes a long time, and the eventual revision surgery is difficult.

### Summary of the invention

Based on the methods and implants of prior art, it is an object of the current invention to provide an implant that seals and/or heals a defect in an annulus in an efficient way to provide immediate mechanical reinforcement of the defect and preferably a subsequent tissue integration of the implant and biological repair of the defect.

According to the invention, an implant is provided for sealing and/or healing a defect in an annulus comprising an area intended to encompass the defect. The implant is a textile strip wherein the textile strip comprises:
- a middle area which is intended to encompass the defect and which is an oriented low-density embroidered structure for guided tissue ingrowth and reinforcement, and
- end areas adjacent to the middle area which are a high-density embroidered structure for fixation.

The implant may comprise various shapes, including but not limited to rectangular, circular, elliptical or compound shapes.. The middle area is located adjacent and in between two end areas. The width of the middle area may be equal or it may be unequal to the width of the end area. It is also possible that the end area encompasses the middle area completely.

One end of the implant is attached to a vertebra and/or to an annulus, whereas the other end is connected to another vertebra and/or to the same annulus. Fastening means which are preferably staples, screws, pins, sutures or adhesive agents are used for the attachment.

### Brief description of the drawings

The drawings will be explained in greater detail by means of a description of an exemplary embodiment, with reference to the following figures:
- Fig. 1: shows a human spinal column, with several textile strips attached at least to an intervertebral disc.
- Fig. 2: shows schematically a composition of a textile strip, with fastening means.
- Fig. 3: shows a textile strip with larger flaps according to the present invention.
- Fig. 4: shows a textile patch according to the present invention.
- Fig. 5: shows the textile strip with a hole structure.
- Fig. 6: shows a half of a textile strip with its embroidery.

### Detailed description of the preferred embodiments

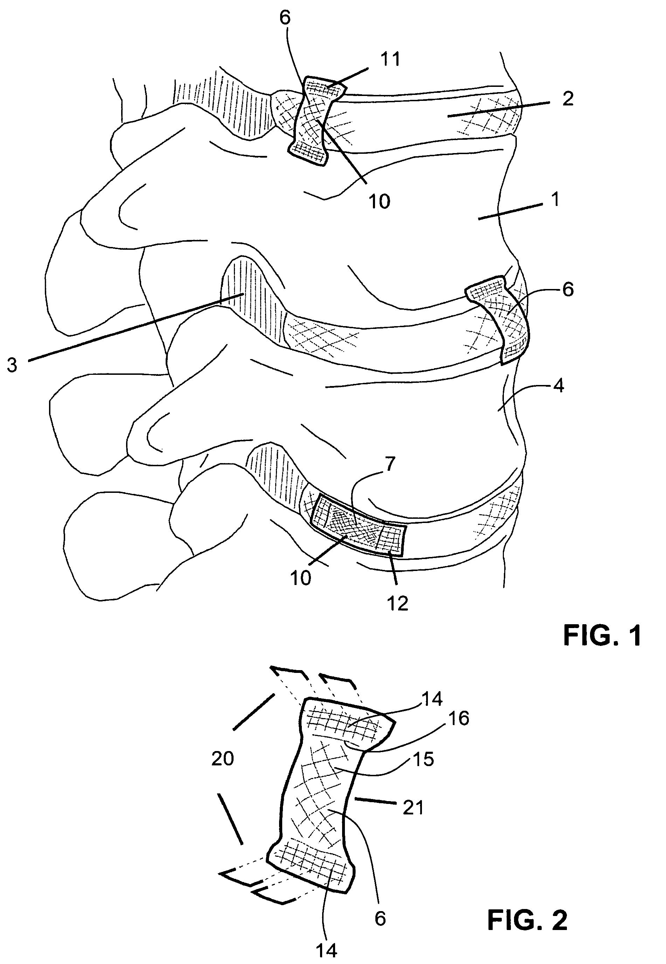

Fig. 1 shows schematically various dispositions of a textile strip 6,7 for sealing and/or healing a defect in an annulus of an intervertebral disc 2 according to the present invention.

The textile strip 6,7, which is also shown in Fig. 2, comprises a middle area 15 and end areas 14. The textile strip 6,7 has substantially a rectangular shape, but may also adopt a circular, elliptical or compound shape. The middle area 15 is arranged between the two end areas 14 and connected to the two end areas 14 by an intermediate area 16. The end areas may also completely encompass the middle area. One edge, normally the longer edge, of the textile strip 6,7 is designated as length, while the other edge is designated as width. However it is possible (and not shown in the drawings) that the width of the textile strip 6,7 is greater than the length.

The middle area 15 is designed to seal and/or heal a defect in the annulus of an intervertebral disc 2 and to provide guidance, whereas the two end areas 14 are designed to be attached to and provide anchorage in the vertebral bodies 1 and/or to the intervertebral disc 2, especially at non-defect places, and to provide reinforcement.

The textile strip 6,7 generally comprises two layers, a base layer and on the base layer a second layer. The base layer is a carrier material and the second layer is an embroidery, made out of a yarn (thread, fibre, filament), which is embroidered onto the carrier material. It is also possible to provide for a textile strip having only a single embroidery layer. The embroidery layer comprises PES Monofil or TREVIRA Monofil, Technora, AE MED or any other suitable yarn or monofil, including natural silk derivatives, and may be impregnated with tissue growth promoting factors. The embroidery layer may consist of a synthetic yarn coated with a layer of silk-derived proteins (fibroin). The carrier material is chosen from any biocompatible textile material. The impregnation of tissue growth promoting factors can be conducted according to WO 2005/019518 and can be applied to the carrier material and/or the embroidered layer. Tissue growth promoting factors can be chosen from the group of linker molecules or activating molecules.

The embroidery in the middle area 15 is an oriented, low-density embroidery. Oriented means that the yarn is embroidered in a determined pattern. The low-density embroidered structure is formed using a light filling stitch structure. After the yarn is embroidered onto the carrier material, the carrier material in the middle area 15 of the textile strip 6,7 may be etched away, thus leaving only the mesh-like textile structure. The carrier material may also be retained to form a heterogeneous, layered construct to promote guided tissue growth on one surface and prevent tissue adhesion on the other. The mesh-like textile structure has a three dimensional surface, an oriented structure, a specific effective pore size and is designed in such a manner, that it is able to reinforce the annulus 2, to provide topographical guidance for tissue growth and to carry tissue growth factors to allow better tissue ingrowth. The effective pore size of the low-density embroidery is approximately 0.5 mm in a prototype device, e.g. between 0.1 and 2 mm, but may also be larger or smaller. The low-density embroidered structure can also be covered with a film, which is an adhesive agent such as described below, in order to attach the middle area 15 to the annulus 2 by an adhesive force.

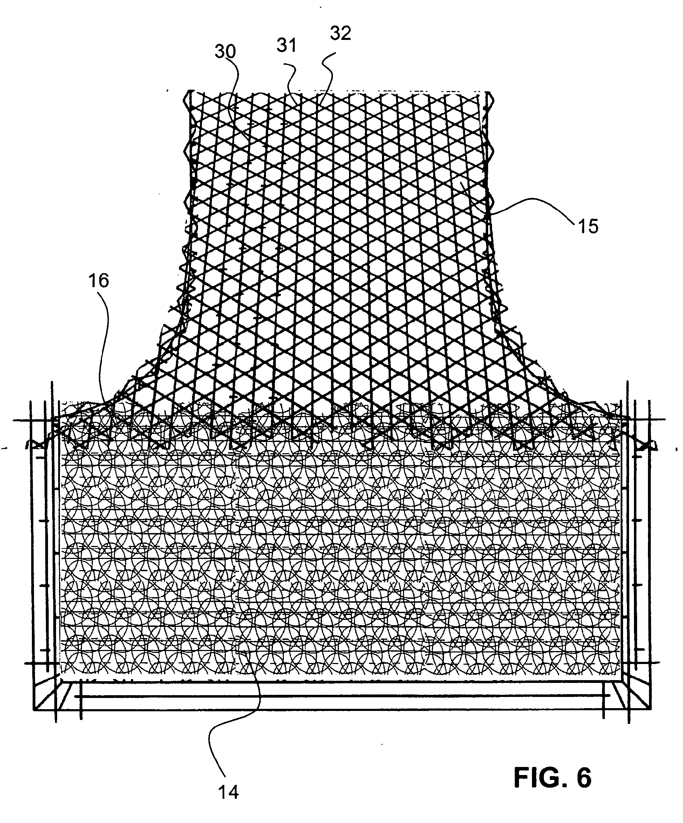

The intermediary area 16, shown schematically in Figure 6, represents a gradient in embroidery density and a continuous transition from the low-density middle area 15 to the high-density end area 14. The intermediate area 16 joins the middle area 15 to the end area 14 with continuous yarn strands incorporated in both areas. The intermediate area 16 with gradient density is typically less than 1 - 2 mm wide, e.g. between 0.4 and 1 mm wide, but may be wider or narrower depending on specific device designs, e.g. up to 5 mm wide.

The movement of the spinal column results in a displacement between two vertebral bodies 1, 4. In order to prevent a forced rupture and maintain the mobility of the spinal column, the textile structure of the middle area 15 has sufficient tensile strength and similar elastic properties to the annulus fibres of the intervertebral disc 2. The tensile modulus of the implant is optimally matched to the properties of the annulus, preferably in the range of 10 - 60 MPa, but may be chosen higher or lower e.g. between 5 and 100 MPa.

In an un-stretched state, the embroidery pattern of the middle area 15 is rectangular and homogeneous, but if the textile structure is stretched lengthwise the cross section of the middle area narrows. The stretched part behaves as the annulus to which it is attached. The middle area may also incorporate non-rectangular lateral extensions. The length and width of the middle area 15 depend on the size and the form of the fissure and on the size of the intervertebral disc 2.

The embroidery in both end areas 14 is a high-density embroidered structure. Both end areas 14 are penetrated or contacted by fastening means 20, which can be staples, pins, sutures, screws or glue. The high-density embroidered structure is formed using a closed filling stitch structure. The effective pore size of the high-density embroidery is approximately 0.2 mm in prototype devices, e.g. between 0.05 and 1 mm, but may also be larger or smaller. The high-density embroidered structure can carry tissue growth-factor material, for bone attachment. The movement of the spinal column not only results in a displacement between two vertebral bodies 1, but also applies very high forces onto the intervertebral disc, which have to be partially carried by the textile strip. The two end areas 14 are responsible to maintain a secure connection between the intervertebral disc 2 and the textile strip, as well as between the vertebral body 1 and the textile strip, therefore sufficient tensile strength is required.

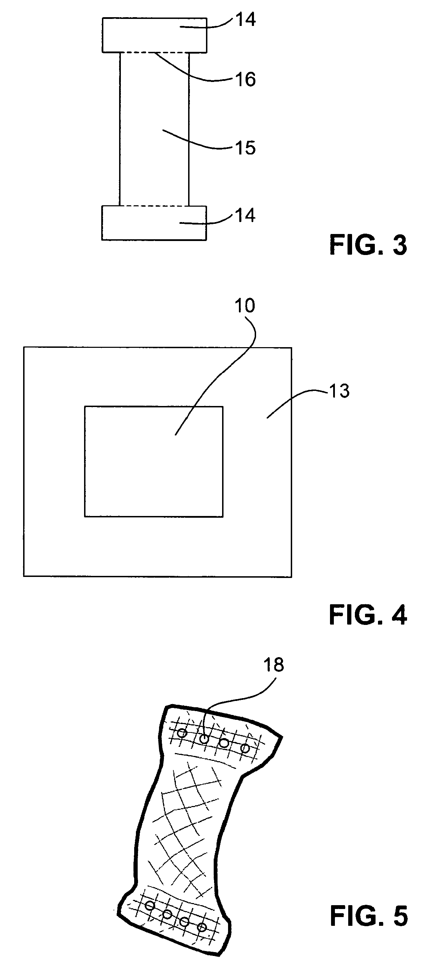

In order to accommodate the fastening means 20, such as pins, staples, sutures or screws, the end area 14 comprises a reinforced hole structure 18 that is adapted to hold staples, pin heads or screw heads. This is schematically shown in Fig. 5. The end area may be further reinforced by incorporating a separate, high-strength yarn. Reinforced regions of the end area may be specifically identified by e.g. colour coding.

The shape of the end areas 14 is preferably rectangular, but may be also trapezoidal or curved to accommodate intervertebral disc or vertebral body geometry. The width of the end area 14 is equal to or greater than the width of the middle area 15 in an un-stretched state. The length of the end area 14 depends on the dimension of the proposed attachment are on the vertebral body or intervertebral disc.

The preferred embodiment shows two arrangements of attaching the textile strip 6,7 either to vertebral bodies 1 or to intervertebral discs 2.

In a first arrangement, shown in Fig. 1, the first end area 14 of the textile strip 6 overlaps a first vertebral body 1 and the second end area 14 overlaps a second vertebral body 4. Both end areas 14 are attached to the vertebral bodies 1,4 by the use of fastening means 20. End areas 14 are preferably attached to the vertebral rim, comprising the high density vertebral endplate and apophyseal ring. Together with the end areas 14 the fastening means 20 are responsible for a safe attachment of the textile strip to the vertebral bodies 1,4. Fastening means may be pins, staples 20, screws, sutures or adhesive agents. Adhesive agents may be cyanoacrylate based adhesives, fibrin sealants, bovine albumin (gluteraldehyde system) and synthetic sealant system (poly ethylene glycol + pol [L-lactide] + poly [trimethylene carbonate]).

If adhesive agents are used, the shear planes of the two end areas have to be designed in order to absorb the corresponding forces applied by the movement of the spinal column.

A second arrangement is also shown in Fig. 1. Both end areas 14 of the textile strip 7 are attached to the intervertebral disc 2. The same fastening means as described in the first arrangement may be used, although adhesive agents are the preferred choice.

The textile strip 6,7 and the fastening means 20, as described above, can be used as a temporary or as a durable measure. The use of biodegradable materials allow that the textile strip and the fastening means is implanted temporarily, dissolving over time.

Fig. 3 shows a further embodiment according to the present invention. The end area 14 shows larger flaps as in the embodiment of Fig 1 and 2.

Fig. 4 shows another embodiment according to the present invention. The high-density embroidered structure 13 shows a rectangular shape. The low-density embroidered structure 10 is located in the centre of the high-density embroidered structure 13, which encompasses the low-density embroidered structure 10 completely. The high-density embroidered structure 13 can be attached to the vertebral body 1 and/or to the intervertebral disc 2 by the use of fastening means as already described.

Fig. 6 shows a pattern, of both the end area 14 and the middle area 15. The middle area 14 shows a smaller width than the end area 15, the middle area 14 merges into the end area 15 with continuous embroidered strands, forming a gradient in embroidery density across a transitional or intermediate area 16. The stitches of the embroidery in the end area 14 are arranged in a curve-like manner. In the middle area 15 the embroidery is arranged in a pattern which comprises a yarn 30 that is oriented lengthwise, a further yarn 31 that is oriented angular to the lengthwise orientation and another yarn 32 that is oriented angular in the same manner as the previous yarn 31. This results in a hexagon-like structure, whereas the hexagons are arranged in straight line. Individual yarn strands cross at an angle of approximately 60°. The yarn used in this embroidery may be a yarn or a monofil as already described.

Another possible pattern of the embroidery consists of yarn oriented parallel to, perpendicular to and at 45° to the long axis of the implant.

In order to implant the textile strip, access to the defect disc and adjoining vertebral bodies has to be provided. This is done by surgical methods which are known by a person skilled in the art. In a first step, the first end of the textile strip 6,7 is attached to the vertebra 1 and/or the annulus 2 . In a further step, the second end of the textile strip 6,7 is attached to another vertebra and/or the annulus. As a last step, the middle area 15 of the implant 6,7 is attached onto the defect in the annulus 2. It is also possible to attach the middle area 15 before the two end areas, that means the steps as described above are not in a particular order.

### Reference Numerals

- 1: Vertebral body
- 2: Annulus / Intervertebral disc
- 3: Spinal canal / Foramen
- 4: Vertebral body
- 6: Textile strip
- 7: Textile strip
- 10: Low-density oriented embroidered structure for tissue in-growth and guidance
- 11: High-density embroidered structure for reinforcement and bone attachment
- 12: High-density embroidered structure for reinforcement and attachment to annulus
- 13: High-density embroidered structure
- 14: End area
- 15: Middle area
- 16: Intermediate area
- 18: Hole structure
- 20: Fastening means
- 21: Primary soft tissue fixation by surgical tissue adhesives
- 30: Lengthwise oriented yarn
- 31: Angular oriented yarn
- 32: Angular oriented yarn

## Claims

1. Implant (6,7) for sealing and/or healing a defect in an annulus of an intervertebral disc (2) comprising an area intended to encompass the defect, **characterized in that** the implant is a textile strip, wherein the textile strip comprises:
- a middle area (14) which is intended to encompass the defect and which is an oriented low-density embroidered structure (10) for guided tissue ingrowth and reinforcement, and
- an end area (15) adjacent to the middle area (14) which is a high-density embroidered structure (11,12,13).

2. Implant (6,7) according to claim 1, wherein the textile strip comprises preferably a rectangular shape and the middle area (14) is located adjacent and in between two end areas (15) and wherein the width of the middle area (14) is equal or unequal to the width of the end area (15).

3. Implant (6,7) according to claim 1, wherein the middle area (15) is completely surrounded by the end area (14), which is preferably rectangular or circular or elliptical.

4. Implant (6,7) according to claims 1 to 3 comprising fastening means (20), which are preferably staples, screws, pins, sutures or adhesive agents, usable to attach one end of the textile strip to a vertebra (1) and/or to an annulus (2) and the other end of the textile strip (11,12,13) to another vertebra (1) and/or to the annulus (2).

5. Implant (6,7) according to claims 1 to 3, wherein the high-density embroidered structure comprises a carrier material and an embroidered structure.

6. Implant (6,7) according to claims 1 to 3, wherein the carrier material of the low-density embroidered structure is etched away, so that only the low-density embroidered structure remains.

7. Implant (6,7) according to claims 1 to 3, wherein the carrier material of the low-density embroidered structure is maintained to provide a heterogeneous, layered construct.

8. Implant (6,7) according to claims 1 to 3, wherein the low-density and/or high-density embroidered structure is composed of a synthetic yarn coated with a layer of silk-derived fibroin protein.

9. Implant (6,7) according to claims 1 to 3, wherein the low-density and/or high-density embroidered structure carries tissue growth-factor material, especially chosen from the group of linker molecules or activating molecules.

10. Implant (6,7) according to claims 1 to 3, wherein the low-density embroidered structure is attached to the annulus by an adhesive agent and/or sutures.

11. Implant (6,7) according to any of the preceding claims, wherein the low-density embroidered structure (10) has similar directional and elastic properties as the annulus of an intervertebral disc (2) of a mammal.

12. Implant (6,7) according to any of the preceding claims, wherein all the materials used are biodegradable.

13. Method to seal and/or heal a defect in an annulus of an intervertebral disc (2) using an implant (6,7) according to one of claims 1 to 12 comprising the steps, executed in any order, of:
- attaching a first end of the implant (6,7) to the annulus (2) and/or the vertebra (1),
- attaching a second end of the implant (6,7) to the annulus and/or to another vertebra (1),
- gluing the middle area (15) of the implant (6/7) onto the defect in the annulus.
